# EUROPEAN PATENT SPECIFICATION

(11) **EP 1 038 286 B1**
(45) Date of publication and mention of the grant of the patent: **03.04.2002**
(21) Application number: 98930865.5
(22) Date of filing: 22.06.1998
(51) Int. Cl.: G09B 23/32, G09B 23/28, A61F 2/38

(54) **ARTIFICIAL JOINTS**
KÜNSTLICHE GELENKE
ARTICULATIONS ARTIFICIELLES

(30) Priority: 24.06.1997 GB 9713186
(43) Date of publication of application: 27.09.2000
(73) Proprietor: THE UNIVERSITY OF SHEFFIELD, Sheffield, S10 2JF (GB)
(72) Inventor: HOLLANDS, Robin, John, Sheffield S6 2RA (GB); McCARTHY, Avril, Dawn, Sheffield S11 7LT (GB); HARLEY, Peter, John, Univ. of Sheffield, Sheffield S3 7RH (GB)
(74) Representative: Allen, Philippa Margaret
(86) International application number: GB9801692
(87) International publication number: WO9859332

(56) References cited:
- GB-A- 2 204 175
- US-A- 4 433 961
- US-A- 5 018 977
- US-A- 5 336 266
- DATABASE INSPEC INSTITUTE OF ELECTRICAL ENGINEERS, STEVENAGE, GB Inspec No. 5131798, TAMARAPALLI J R ET AL: "A multipurpose endoscopy training apparatus" XP002078345 & PROCEEDINGS OF THE 1995 FOURTEENTH SOUTHERN BIOMEDICAL ENGINEERING CONFERENCE (CAT. NO.95TH0703-9), PROCEEDINGS OF THE 1995 FOURTEENTH SOUTHERN BIOMEDICAL ENGINEERING CONFERENCE, SHREVEPORT, LA, USA, 7-9 APRIL 1995, pages 297-299, ISBN 0-7803-2083-2, 1995, New York, NY, USA, IEEE, USA

## Description

The invention relates to an artificial joint which is designed to mimic the movement of a selected human or animal joint and which is further provided with a cavity into which selected instruments can be placed and then manipulated without obstruction.

The invention is for use particularly, but not exclusively, as part of a computer simulation training system whereby surgeons can be taught the necessary navigational and triangulation skills that are required to perform arthroscopic surgery successfully.

Arthroscopy is a form of endoscopy concerned specifically with joints. Endoscopy is a surgical technique for operating on a region within the body whilst minimising the damage to skin, muscle, surrounding tissues and organs. Because of this, it is referred to commonly as minimally invasive or "keyhole" surgery. Performed by skilled surgeons, it causes less disruption than open surgery and usually results in improved patient recovery times. In England alone, during 1994/5, 28,530 primary arthroscopies of the knee were performed (Department of Health figures). Approximately 60% of these cases were performed as day cases, thereby reducing the associated costs of patient intervention.

In arthroscopy, unlike open joint surgery, the surgical environment is effectively remote although the surgeon is situated next to the patient. The internal view of the joint is achieved by introducing an arthroscope into the joint through a small skin incision. Surgical instruments are introduced through secondary skin incisions. The arthroscope is a rigid instrument consisting of a fibre optic camera and light source. The camera relays images of the joint to a video monitor that is viewed by the surgeon during surgery. The view displayed, unlike that during open surgery, is usually monoscopic rather than stereoscopic, and thus perceiving depth is more difficult for the surgeon. The manipulations of the instruments are more limited because the entry portals into the joint constrain the usual number of degrees of freedom from six to four. Thus, the surgeon must learn new strategies for navigating the arthroscope and instruments within the joint. To complicate the learning process further, the arthroscope used most commonly in knee surgery has a 30 offset whereby the view shown is at an angle of 30 to the long axis of the arthroscope.

During a joint inspection, the line of sight of the arthroscope may be obstructed by internal joint structures. To improve the image seen, the surgeon must manipulate the joint to open up tight spaces within, and so allow the arthroscope a clearer view. Competence at navigating within the joint involves the surgeon co-ordinating the movements of the arthroscope with those of the joint, while avoiding contact by the arthroscope with the articular surfaces.

In addition to gaining competence at navigation, a key requirement of arthroscopy is the ability of the surgeon to triangulate, or guide, an instrument introduced through a different portal into the field of view of the arthroscope. Again, once this skill has been acquired, the surgeon must co-ordinate manipulations of the joint with triangulation of the surgical instruments, while minimising joint damage by avoiding contact with joint surfaces.

It has been reported by expert arthroscopic surgeons that it may take more than 500 and probably closer to 800 procedures before diagnostic proficiency is reached (Miller, 1985). Diagnostic proficiency can only be achieved once navigational competence has been gained.

Because arthroscopy requires a level of three dimensional perception and hand-eye co-ordination much greater than that required for traditional surgery, and because supervised surgery is time consuming, expensive and potentially hazardous to the patient, attempts have been made to create training systems for arthroscopic surgeons.

Although animals are used for training in some countries, their use is prohibited at least in the U.K. An alternative would be to use human cadaver limbs, but these can be very difficult to obtain and are subject to ethical and as well as fixation considerations.

In the U.S.A. a simple mount has been designed to allow mounting a cadaveric lower limb for the purpose of arthroscopic training (Fullerton et al., 1981). Inclusion of the hip is not provided for in the design of the stand and so the possible manipulations are limited to those of the knee. The choice of cadaver limb is also limited, to either fresh or thawed fresh frozen limbs; chemically fixed tissues will display altered kinematic and visual properties compared with fresh tissues.

Typically, for knee arthroscopy, simple physical models (comprising replica bones and soft tissue structures covered in latex), see, for example GB 2 204 175, are used for initial training in conjunction with real arthroscopic cameras. These simple models are considered by surgeons to be unrealistic and not sufficiently testing of technique. The models do not allow for realistic joint manipulation, and their components are crude in design and require repair-work after training sessions. The use of real cameras risks damage to expensive equipment.

Another approach has been to focus on the general skills, of hand-eye co-ordination and precise movements, required for arthroscopy. One example is the "black box" technique employed by Meyer et al. (1993) which used a 6 x 6-inch box to enclose a model femur, anterior cruciate ligament and a piece of brass wire shaped to simulate a meniscus. It provided a clinically relevant space into which the instruments could be placed but made no attempt to mimic a lower limb.

A multimedia trainer for shoulder arthroscopy has been produced using a large database of video footage. (Cooper et al., 1995; Grange et al., 1996). Its use is restrictive in that only the scenes stored within the database can be displayed. The trainer does not incorporate a physical model of the relevant joint i.e. the shoulder with which the surgeon can interact; the patient having been replaced by "a half melted slab of butter- or a more appropriate material" (Cooper et al., 1995, p21). Thus the scope and the realism of the video-based shoulder arthroscopy trainer is limited.

Alternative, more realistic methods of training have therefore been sought. With the continuing development of computers, virtual reality (VR) technology has provided the means by which training systems for arthroscopic surgeons can be constructed. A number of VR systems have been created (Logan et al., 1996; Ziegler et al., 1995). However, Logan et al. (1996) have not provided a limb in their system preferring to concentrate on realistic computer graphics. An important feature of arthroscopy, i.e. joint manipulation, has thus been disregarded. The system created by Ziegler et al. (1995) does provide a physical knee model, but, the properties of the model have not been described. However, the hip, and thus the degrees of freedom provided by the hip joint have not been incorporated within the model.

We consider that traditional physical models used currently in arthroscopy training cannot serve as input devices for VR training systems, while cadaver limbs are unsuitable because of ethical considerations. This is because neither traditional physical models nor cadaver limbs provide a space into which the arthroscope and instruments can be inserted and manipulated without physical constraint. Since the images of the joint are created by computer software and are dependent on input provided by the instrument sensors, any physical obstruction of the instruments would prevent suitable images being created. Therefore, in a VR arthroscopy system, we consider physical replication of internal structures within a joint is undesirable and unnecessary.

It is therefore an object of the invention to provide an artificial joint for use particularly but not exclusively, in a VR training system.

According to a first aspect of the invention there is therefore provided an artificial joint adapted to connect theretogether at least two members wherein said joint is fashioned so as to mimic the movement of a selected human or animal joint and also so as to provide a cavity into which at least one selected instrument can be placed and then manipulated without obstruction.

In a preferred embodiment of the invention said two members are arranged for relative movement without obstructing the site(s) whereat said selected instrument is/are inserted and also without obstructing the area of the joint in which manipulation of said selected instrument is to take place thus allowing for free manipulation of said selected instrument.

In a yet further preferred embodiment of the invention said artificial joint comprises a hinge mechanism between said members and ideally said hinge mechanism provides for rotation about at least two axes and preferably about a primary axis and a secondary axis. Ideally said primary and said secondary axis are orthogonal.

More preferably further still, said artificial joint comprises a relatively rigid coupling connected at at least one site to a first of said members and at least one other site to a second of said members via a non-direct route i.e. that is to say a route that does not obstruct said cavity in which said instrument is manipulated. For example, said non-direct route may take the form of a curve designed to circumvent said cavity. Clearly, the nature of the curvature will be designed so as to circumvent said cavity, as aforementioned, but also so as not to interfere with the manipulation of said members. More ideally still, said artificial joint comprises a first U-shaped member attached at a first end to a first position and at a second end to a second, ideally opposite, position on a first of said members; and a second, similarly U-shaped member attached at a first end to a first position and at a second end to a second, ideally opposite, position on a second of said members. More preferably still, said first and said second U-shaped members are attached theretogether or integral therewith and ideally relatively positioned so as to be at right angles with respect to each other, thus defining a cross-shaped member of hemi-spherical nature.

As previously, mentioned, said artificial joint is ideally attached to said members at suitable sites so as to ensure that the site(s) where said instruments is/are to be inserted are left clear. Moreover, said insertion site(s) may be covered with a suitable material so as to provide a continuous surface from one of said members to the other. Ideally, this material is selected so as to be of an appropriate thickness either for the purpose of permitting easy insertion and thus learning surgical entry points, or thick enough to provide a more realistic resistance to manipulation of the devices around entry portals.

In yet a further preferred embodiment of the invention said artificial joint is provided with means to govern the relative movement of said members and ideally is provided with means for determining movement along or about at least one selected axis, which means includes limiting means for limiting the degree of movement along or about said axis, for example, means for limiting the degree of respective rotation of a member about an axis. Preferably, said limiting means may comprise mechanical or electro-mechanical means, for example, said limiting means may comprise suitably positioned stop members and/or cam dependent movement devices. Alternatively, or in addition, said limiting means may comprise electromechanical means to allow electronic control of movement and ideally rotational limits, for example, electromechanical servo mechanisms may be employed.

In a yet further preferred embodiment of the invention said artificial joint and/or said associated members are provided with at least one sensor for monitoring the relative position of one member with respect to said other member and for conveying this information to a VR system whereby the relative movement of said members results in a corresponding change in VR images so as to simulate the images that one would normally view during a real surgical procedure.

According to a further aspect of the invention there is provided an artificial limb including the artificial joint according to any proceeding embodiment.

An embodiment of the invention will now be described by way of example only with reference to the following figures wherein:

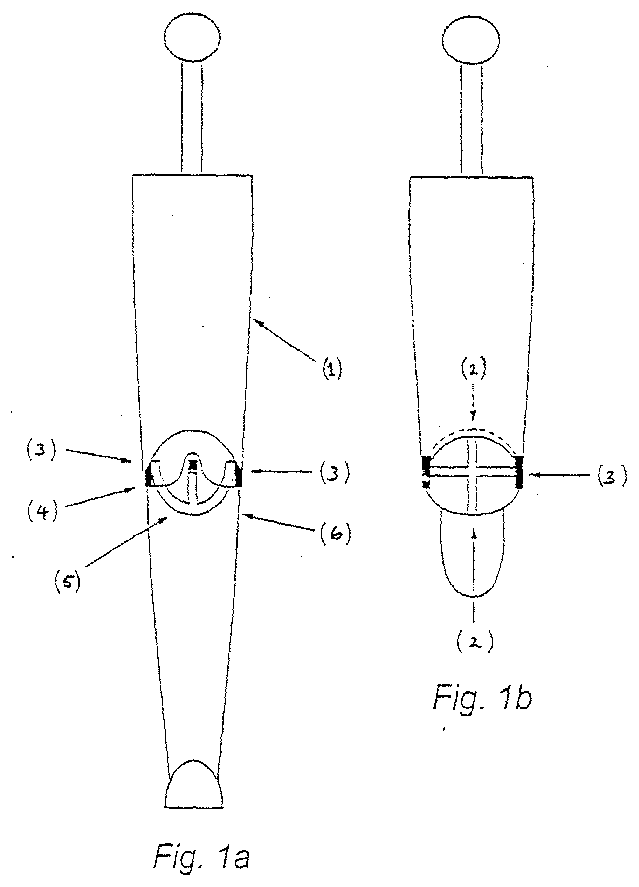

Figure 1 shows a front view of an artificial knee joint in extended, Figure 1a, and flexed, Figure 1b, positions wherein (1) is a upper leg primary limb shell; (2) is a secondary axis; (3) is a primary axis; (4) is a secondary axis rotation limiting cam; (5) is a linkage mechanism; (6) is a lower leg secondary limb shell; and (2) is a secondary axis:

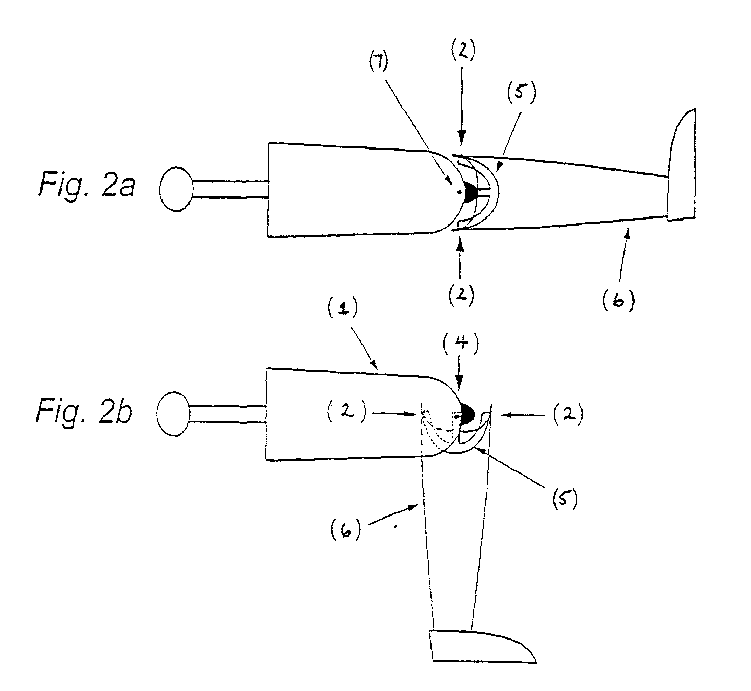

Figure 2 shows a side view of the artificial knee joint shown in Figure 1 in extended, Figure 2a, and flexed, Figure 2b, positions wherein (7) is a secondary axis rotation limiting cam: (2) is a secondary axis; (5) is a linkage mechanism; (1) is a upper leg primary limb shell; (2) is a secondary axis; (6) is a lower leg secondary limb shell; (4) is a secondary axis rotation limiting cam; (2) is a secondary axis; (5) is a linkage mechanism; and (6) is a lower leg secondary limb shell;

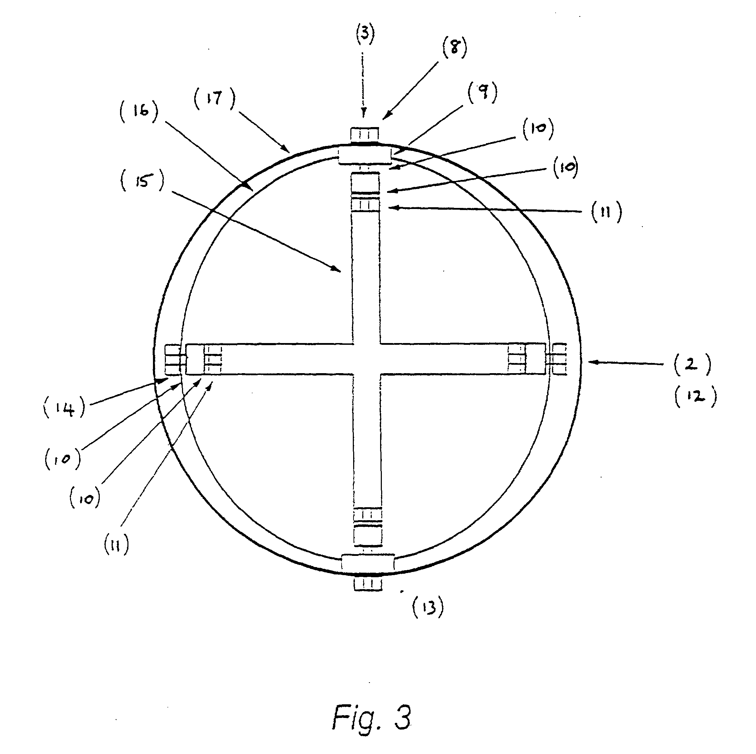

Figure 3 shows a transverse sectional view of the knee joint shown in Figures 1 and 2 wherein (3) is a primary axis; (8) is a primary axis pivot bolt; (9) is a secondary rotation limit cam; (10) is a slip washer; (11) is a lock nut; (2) is a secondary axis; (12) is a secondary axis pivot mechanism; (13) is a primary axis pivot mechanism; (11) is a lock nut; (14) is a secondary axis pivot bolt; (15) represents a physical linkage; (16) is a secondary limb shell; and (17) is a primary limb shell;

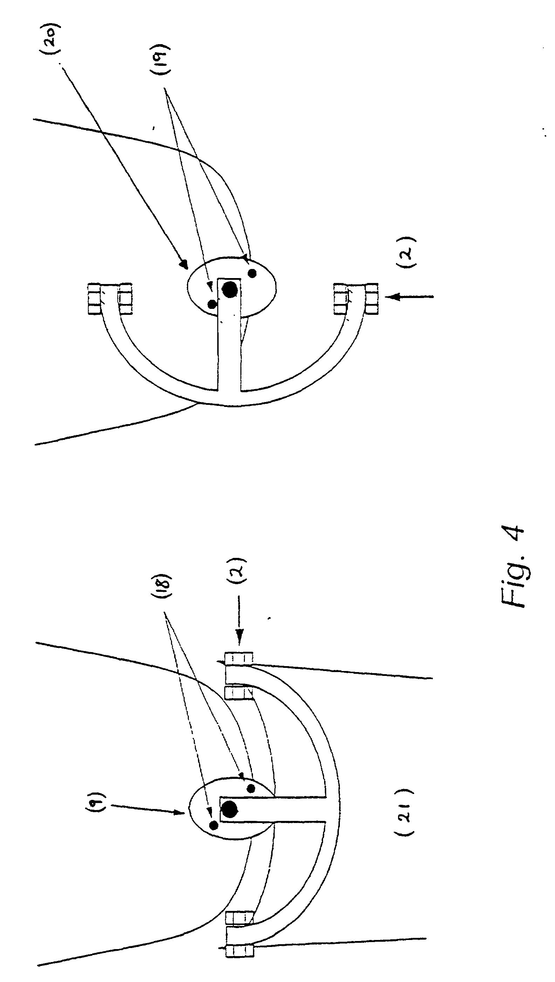

Figure 4 shows a side view of an artificial knee joint having means for limiting rotation of the joint about a first axis wherein (9) is a secondary rotation limit cam (no secondary rotation possible with no primary rotation, in this case); (18) primary axis rotation limits stops; (19) primary axis limits stops; (20) is a secondary rotation limit cam (some secondary rotation possible with primary rotation, in this case; (2) is a secondary axis; cam permits some secondary axis rotation at primary axis rotation B; and (21) cam restricts secondary axis rotation at primary axis rotation A;

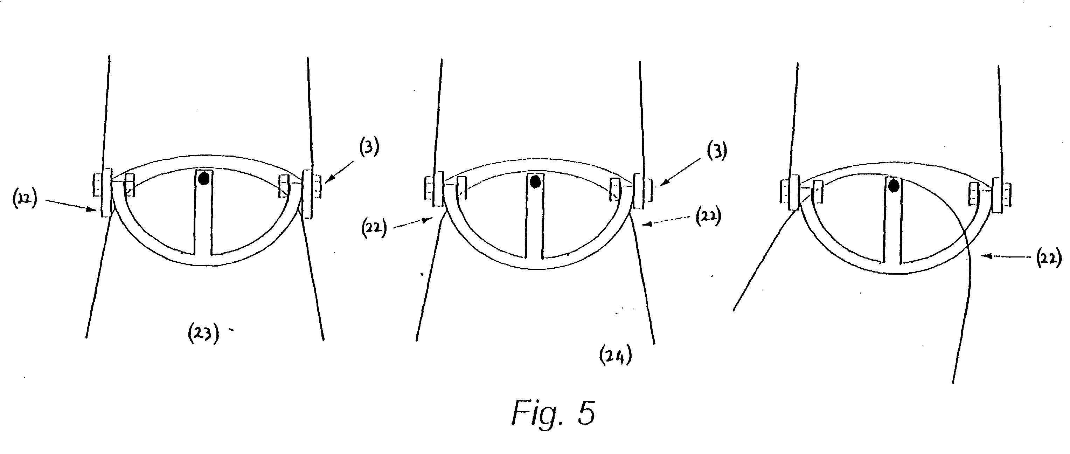

Figure 5 shows a front view of an artificial'knee joint having means for limiting rotation of the joint about a second axis wherein (3) is a primary axis; (22) is a contact point; (23) limiting cams are contacting both contact points, no secondary axis rotation is possible; and (24) any gap between a cam and a contact point allows secondary axes rotation until contact is made;

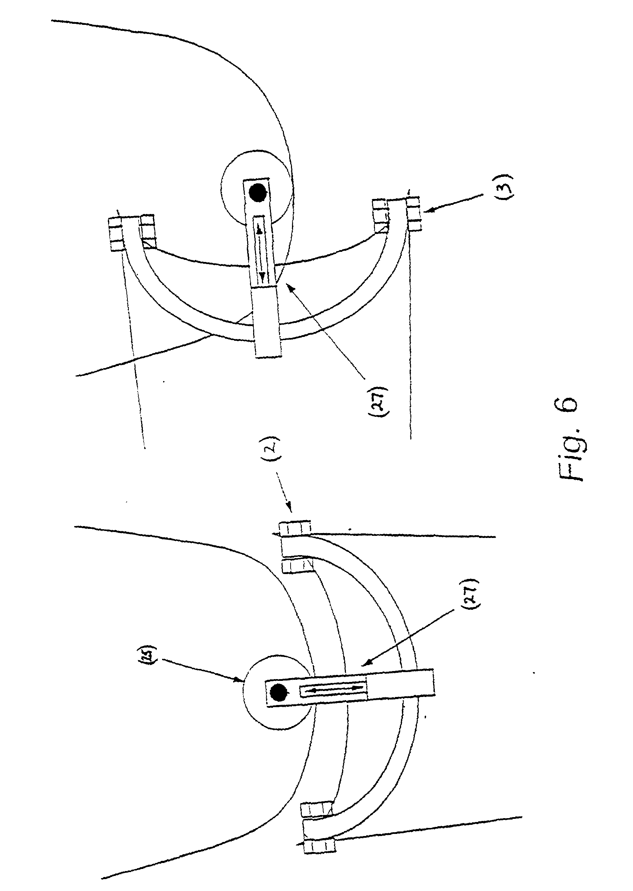

Figure 6 shows a side view of a yet alternative embodiment of the invention including electromechanical means for limiting rotation about a secondary axis wherein (25) is a circular cam; (2) is a secondary axis; and (27) linear servo extension is varied to control the degree of possible secondary rotation;

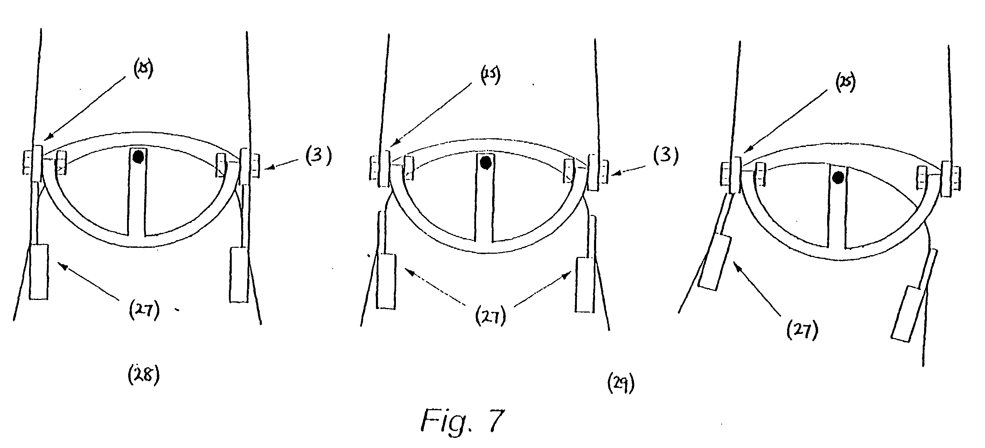

Figure 7 shows a front view of a knee joint including an alternative means for limiting secondary axis rotation wherein (25) is a circular cam; (3) is a primary axis; (27) are secondary axes rotation limiting servos; (28) if secondary axes rotation limiting servos are extended to contact both cams, no secondary axis rotation is possible; and (29) if secondary axes rotation limiting servo is withdrawn to provide a gap between the actuator and the cam, secondary axes rotation is possible until contact is made; and

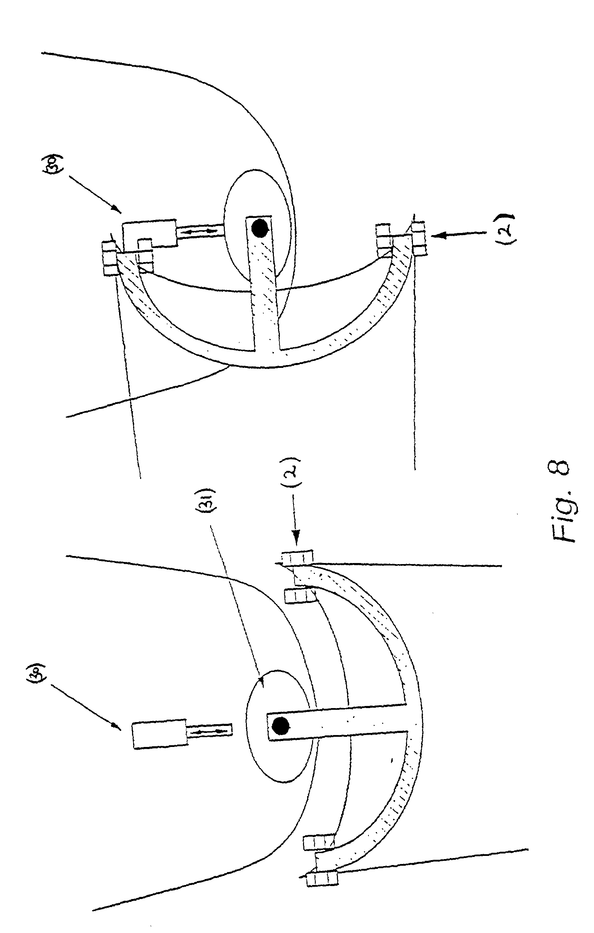

Figure 8 shows a side view of a knee joint with a further alternative means for limiting primary axis rotation wherein (30) linear servo extension is set to control the amount of primary rotation possible; (31) cam has increasing radius from zero point to the outer limits of primary axis rotation; and (2) is a secondary axis.

Referring now firstly to Figures 1 and 2, it can be seen that a physical linkage (5) is designed to provide a hinge mechanism between two physical shells (1,6) representing limb areas either side of the simulated joint (e.g. the upper (1) and lower (6) leg, for a simulated knee joint, see Figure 1 and Figure 2). The limb shell which rotates around the primary axis (3) of the physical linkage (5) is referred to as the primary limb shell (1) (whereas the limb shell which rotates around the secondary axis (2) of the physical linkage (5) is referred to as the secondary limb shell (6). The mechanism provides two orthogonal rotational axes without the obstruction within the central operating area which would result if a simple straight hinge was used. This is achieved by using a rigid linkage which links the two limb shell attachment points on each axis, by using a non-direct route, typically a curve into the non-instrument-accessible area of one of the limb shells (1) and (6). The linkages for each axis are connected at at least one point to provide the appropriate rigid coupling (5). The curvature of the route between attachment points varies according to the particular joint being simulated, but must be greater than that required to leave the necessary central operating area clear, but smaller than that which would cause the rotation of the mechanism to be obstructed by the linkage (5) coming into contact with the limb shell, within the rotation limits required by the joint model. Some form of pin joint (8), such as a simple bolt, is used to attach the limb shell to its attachment point pair on the linkage (8), in order to allow the shell to rotate around the appropriate axis, see Figure 3. The area of the limb shells around the joint must be shaped such that the surface area where devices may be inserted into the joint is left clear, and also such that the structure of one shell does not obstruct the movement of the other within the rotational limits expected from the joint. An elastic material is used to provide a continuous surface from one limb shell to the other, and covers the void area left around the physical linkage (5). This elastic material may be thin enough to permit easy incision, for the purpose of learning surgical entry points, or of a thicker material, to provide more realistic resistance to manipulation of the devices around any entry portals.

Since the invention is intended primarily as an input device for computer simulations, it is necessary to provide an electronic method of monitoring joint position. This can be achieved by the use of a passive rotational sensor, such as a potentiometer, on each axis, typically at its attachment point with the limb shell. Where the devices, such as replica instruments, being used within the knee are being monitored actively, e.g. by the use of electromagnetic sensors, the same technology can be used to monitor the position of the lower limb (6) with respect to the upper limb (1), by placing the transmitter unit in one, and the sensor in the other.

Typically, rotation around the primary axis (3) will have fixed limits independent of the secondary axis (2), and can be mechanically limited by the use of physical stops to the linkage (5), obstructing further rotation around that axis, see Figure 4. If rotation around the secondary axis, (2) is also fixed and independent of the rotation around the primary axis (3), a similar system of physical stops may be used.

Where the limits of rotation around the secondary axis (2) are a function of the current rotation around the primary axis (3), e.g. the flexion dependent nature of abduction-adduction in the knee, a cam mechanism (10) is used. The two cams (9) are fixed to the primary limb shell (1), with each centred around the one of the two linkage attachment points on the primary shell, see Figure 4. The radius of the cam would typically vary with primary axis angle to allow varying degrees of rotation of the secondary axis (2) with respect to the primary axis rotation (3), see Figure 5. The cams (9) should be of the appropriate size and position that one would just come into contact with an area on the secondary shell (6) when the secondary shell (6) has been rotated around the secondary axis (2) by an amount equal to the maximum desired rotation at that point. The two cams (9) may have different radial profiles to allow non-symmetric rotation limits around the secondary axis (2).

An extension of the mechanical rotational limit method, to allow electronic control of the rotational limits, for example to reflect the physical effect of a simulated pathology, is to use electromechanical servo mechanisms. Whereas the mechanical method uses a fixed contact point on the secondary shell (6) with a cam whose profile varies with primary axis angle, the electromechanical method utilises a circular cam (25), but varies the position of the contact point by the use of a linear servo-mechanism (27, 29), see Figure 7. The possible rotation around the secondary axis (2) is then limited by the current extension of the servo-mechanism, see Figure 7, and would normally be controlled as a function of the primary axes rotation (3) measured electronically as described above. As with the mechanical method, the two linear servo mechanisms (27) may be set to different values to allow for non-symmetric rotation limits around the secondary axis (2).

A similar system can be used to set rotational limits around the primary axis (1) by using a primary-shell-mounted linear servo-mechanism ( 30) in conjunction with a primary-axes-mounted cam (31), attached to or incorporated into the physical linkage (5), whose radius gets larger as the primary rotational angle varies from a joint specific centre point, see Figure 8. Primary angle rotation limits are then determined by the point at which the linear servo-mechanism (30) comes into contact with a cam. Where the ratio between the positive and negative rotation limits of the primary axis remain fixed, it is possible to use a single 'double ellipse' shaped cam. However, where separate control over positive and negative primary rotation limits is required, a simpler cam profile can be used to restrict rotation only in one direction. In this case, a second servo and cam is used at the other shell attachment point on the primary axis to restrict rotation in the other direction.

It can therefore be seen that the invention concerns the provision of an artificial joint with a view to providing a manipulable member for use in a virtual reality training system.

### References

Cooper J., Ford L. and Watson G. (1995)
Video worlds: a training potential for non-invasive surgery

Research Report 336, ftp:/ftp dcs.ex.ac/pub/usr/lindsey/garth.ps.Z pp1-21

Fullerton L.R., Protzman R.W. and Wincheski J. (1981)
Arthroscopy Training
The American Journal of Sports Medicine **9**:1:38-39

Grange S., Bunker T. and Cooper J. (1996)
Networking virtual reality for shoulder arthroscopy
The British Journal of Healthcare Computing & Information Management 13:10:26-28
Logan I.P., Wills D.P.M., Mohsen A.M.M.A. and Sherman K.P. (1996)
Virtual Environment Knee Arthroscopy Training System
Proceedings of Simulation in Synthetic Environments '96 and 1996 Simulation for Emergency Management Proceedings of the 1996 Simulation Multiconference
April 8-11 1996, New Orleans, Louisiana, p11-16

Miller W.E. (1985)
Learning Arthroscopy
Southern Medical Journal 7 **8**:8:935-940

Meyer R.D., Tamarapalli J.R. and Lemons J.E. (1993)
Arthroscopy training using a "black box" technique
Arthroscopy : The journal of arthroscopic and related surgery **9**:3:338-340

Ziegler R., Fischer G., Müller W. and Göbel M. (1995)

Virtual Reality Arthroscopy Training Simulator.
Computers in Biology and Medicine **25**:2:193-203

## Claims

1. An artificial joint adapted to connect at least two members (1) and (6) theretogether wherein said joint is fashioned so as to mimic the movement of a selected human or animal joint and also to provide a cavity into which at least one selected instrument can be placed and then manipulated without obstruction.

2. An artificial joint according to Claim 1 wherein said members (1) and (6) are arranged for relative movement without obstructing the site(s) whereat said selected instrument(s) is/are inserted and also without obstructing the area of the joint in which manipulation of said selected instrument is to take place thus allowing for free manipulation of said selected instrument.

3. An artificial joint according to Claims 1 or 2 wherein said artificial joint comprises a hinge mechanism (5) between said members (1,6).

4. An artificial joint according to Claim 3 wherein said hinge mechanism provides for rotation about at least two axes (2) and (3).

5. An artificial joint according to Claim 4 wherein one of said axes is a primary axis (3) and a second of said axes a secondary axis (2).

6. An artificial joint according to Claim 5 wherein said primary and secondary axes (2) and (3) are orthogonal.

7. An artificial joint according to Claims 1-6 wherein said artificial joint comprises a relatively rigid coupling (5) connected at at least one site to a first of said members and at least one other site to a second of said members via a non-direct route.

8. An artificial joint according to Claim 7 wherein said coupling comprises a curved configuration.

9. An artificial joint according to Claims 7 or 8 wherein said coupling comprises a first U-shaped member attached at a first end to a first position and at a second end to a second, opposite position, on a first of said members; and a second, similarly U-shaped member attached at a first end to a first position and at a second end to a second, opposite position, on a second of said members.

10. An artificial joint according to Claim 9 wherein said second U-shaped members are attached theretogether or provided as an integral unit (5).

11. An artificial joint according to Claim 10 wherein said members are positioned so as to be at right angles with respect to each other, thus defining a cross shaped member of hemispherical nature (5).

12. An artificial joint according to any preceding Claim wherein said joint is at least partially covered with material.

13. An artificial joint according to Claim 12 wherein said material covers said joint so as to provide a continuous surface from one of said members (1) to the other of said members (6).

14. An artificial joint according to Claims 12 or 13 wherein said material is selected so as to be of a thickness appropriate for suitable manipulation of said instrument.

15. An artificial joint according to Claims 1-14 wherein said artificial joint is provided with means (25,27; 30,31,9,18,19;20) to govern the relative movement of said members.

16. An artificial joint according to Claim 15 wherein said means comprises means for determining movement along or about at least one selected axis (2,3), which means includes limiting means (9,18,19,20;23,27;30,31) for limiting the degree of movement along or about said axis.

17. An artificial joint according to Claim 16 wherein said limiting means comprises mechanical (9,18,19,20) or electro-mechanical (25,27;30,31) means.

18. An artificial joint according to Claims 1-17 wherein said joint is provided with at least one sensor for monitoring the relative position of one member with respect to said other member and for conveying this information to a video recording system whereby the relative movement of said members results in a corresponding change in video recording images so as to simulate the images that one would normally view during surgical procedure.

19. An artificial limb including the artificial joint according to Claims 1- 18.

## Patentansprüche

1. Ein künstliches Gelenk, dass derart angepasst ist, um mindestens zwei Teile (1) und (6) mit diesem zu verbinden, wobei das Gelenk derart gestaltet ist, um die Bewegung eines bestimmten menschlichen oder tierischen Gelenks nachzuahmen und um auch eine Ausnehmung bereit zu stellen, in welche mindestens ein ausgewähltes Instrument gelegt und dann ohne Behinderung manipuliert werden kann.

2. Ein künstliches Gelenk gemäß Anspruch 1, wobei die Teile (1) und (6) vorgesehen sind zur relativen Bewegung ohne Behinderung der Stelle bzw. Stellen, an der bzw. an denen das ausgewählte Instrument bzw. die ausgewählten Instrumente eingeführt werden, und auch ohne Behinderung des Bereichs des Gelenks, in dem die Manipulation des ausgewählten Instruments stattfinden soll, um so eine freie Manipulation des ausgewählten Instruments zu ermöglichen.

3. Ein künstliches Gelenk gemäß einem der Ansprüche 1 oder 2, wobei das künstliche Gelenk einen Scharniermechanismus (5) zwischen den Teilen (1, 6) aufweist.

4. Ein künstliches Gelenk gemäß Anspruch 3, wobei der Scharniermechanismus eine Rotation um mindestens zwei Achsen (2) und (3) gewährleistet.

5. Ein künstliches Gelenk gemäß Anspruch 4, wobei eine der Achsen eine Primärachse (3) und die zweite der Achsen eine Sekundärachse (2) ist.

6. Ein künstliches Gelenk gemäß Anspruch 5, wobei die Primär- und Sekundärachsen (2) und (3) orthogonal sind.

7. Ein künstlich Gelenk gemäß einem der Ansprüche 1-6, wobei das künstliche Gelenk eine relativ steife Kopplung (5) aufweist, die an mindestens einer Stelle mit dem ersten Teil und an mindestens einer anderen Stelle mit dem zweiten Teil über eine nicht direkte Weise verbunden ist.

8. Ein künstliches Gelenk gemäß Anspruch 7, wobei die Kopplung eine gekrümmte Konfiguration aufweist.

9. Ein künstliches Gelenk gemäß Anspruch 7 oder 8, wobei die Kopplung ein erstes u-förmiges Teil, das an einem ersten Ende mit einer ersten Position und an einem zweiten Ende mit einer zweiten, gegenüberliegenden Position an einem ersten Ende des ersten Teils befestigt ist, und ein zweites, auf ähnliche Weise u-formiges Teil aufweist, das an einem ersten Ende mit einer ersten Position und an einem zweiten Ende mit einer zweiten, gegenüberliegenden Position an dem zweiten Teil befestigt ist.

10. Ein künstliches Gelenk gemäß Anspruch 9, wobei die zwei u-förmigen Teile miteinander befestigt sind oder als integrierte Einheit (5) vorgesehen sind.

11. Ein künstliches Gelenk gemäß Anspruch 10, wobei die Teile so positioniert sind, dass sie zueinander in rechten Winkeln stehen, um somit ein kreuzförmiges Teil von halbkugelartiger Form (5) zu definieren.

12. Ein künstliches Gelenk gemäß einem der vorhergehenden Ansprüche, wobei das Gelenk mindestens teilweise mit einem Material umfasst ist.

13. Ein künstliches Gelenk gemäß Anspruch 12, wobei das Material das Gelenk derart umfasst, um eine kontinuierliche Fläche von einem der Teile (1) zu dem anderen Teil (6) vorsieht.

14. Ein künstliches Gelenk gemäß Anspruch 12 oder 13, wobei das Material derart ausgewählt ist, um in seiner Dicke der geeigneten Manipulation des Instruments zu entsprechen.

15. Ein künstliches Gelenk gemäß einem der Ansprüche 1-14, wobei das künstliche Gelenk mit Mitteln (25, 27; 30, 31, 9, 18, 19; 20) vorgesehen ist, um die relative Bewegung der Teile zu regeln.

16. Ein künstliches Gelenk gemäß Anspruch 15, wobei die Mittel Mittel zur Bestimmung von Bewegung entlang und um mindestens eine ausgewählte Achse (2, 3) aufweisen, wobei die Mittel begrenzende Mittel (9, 18, 19, 20; 23, 27; 30, 31) zur Begrenzung des Bewegungsmaßes entlang und um die Achse umfassen.

17. Ein künstliches Gelenk gemäß Anspruch 16, wobei die begrenzenden Mittel mechanische (9, 18, 19, 20) oder elektro-mechanische (25, 27; 30, 31) Mittel aufweisen.

18. Ein künstliches Gelenk gemäß der Ansprüche 1-17, wobei das Gelenk mit mindestens einem Sensor zum Überwachen der relativen Position eines Teils bezüglich des anderen Teils und zum Übermitteln dieser Information an ein Videoaufnahmesystem vorgesehen ist, wobei die relative Bewegung der Teile in einer entsprechenden Änderung in den Videoaufnahmebildern resultiert, um so die Bilder zu simulieren, die man normalerweise während eines chirurgischen Eingriffs sehen würde.

19. Ein künstliches Glied, das ein künstliches Gelenk gemäß der Ansprüche 1-18 umfasst.

## Revendications

1. Articulation artificielle conçue pour relier au moins deux éléments (1) et (6) ensemble, dans laquelle ladite articulation est configurée de façon à imiter le mouvement d'une articulation humaine ou animale sélectionnée et pour procurer également une cavité dans laquelle au moins un instrument sélectionné peut être placé et ensuite manipulé sans obstruction.

2. Articulation artificielle selon la revendication 1, dans laquelle lesdits éléments (1) et (6) sont disposés en vue d'un mouvement relatif sans obstruer le ou les sites au niveau desquels le ou les instruments sélectionné (s) est/sont inséré(s) et également sans obstruer la région de l'articulation dans laquelle la manipulation dudit instrument sélectionné doit avoir lieu en permettant ainsi une manipulation libre dudit instrument sélectionné.

3. Articulation artificielle selon les revendications 1 ou 2, dans laquelle ladite articulation artificielle comprend un mécanisme de charnière (5) entre lesdits éléments (1, 6).

4. Articulation artificielle selon la revendication 3, dans laquelle ledit mécanisme de charnière permet une rotation autour d'au moins deux axes (2) et (3).

5. Articulation artificielle selon la revendication 4, dans laquelle l'un desdits axes est un axe primaire (3) et un second desdits axes est un axe secondaire (2).

6. Articulation artificielle selon la revendication 5, dans laquelle lesdits axes primaire et secondaire (2) et (3) sont orthogonaux.

7. Articulation artificielle selon les revendications 1 à 6, dans laquelle ladite articulation artificielle comprend un accouplement relativement rigide (5) raccordé au niveau d'au moins un premier site à un premier desdits éléments et d'au moins un autre site à un second desdits éléments par l'intermédiaire d'une voie non directe.

8. Articulation artificielle selon la revendication 7, dans laquelle ledit accouplement comprend une configuration incurvée.

9. Articulation artificielle selon la revendication 7 ou 8, dans laquelle ledit accouplement comprend un premier élément en forme de U fixé au niveau d'une première extrémité à une première position et au niveau d'une seconde extrémité à une seconde position opposée, sur un premier desdits éléments, et un second élément en forme de U de façon semblable fixé au niveau d'une première extrémité à une première position et au niveau d'une seconde extrémité à une seconde position opposée, sur un second desdits éléments.

10. Articulation artificielle selon la revendication 9, dans laquelle lesdits seconds éléments en forme de U sont fixés ensemble ou sont réalisés sous forme d'une unité intégrée (5).

11. Articulation artificielle selon la revendication 10, dans laquelle lesdits éléments sont positionnés de façon à être à angle droit l'un par rapport à l'autre, en définissant ainsi un élément en forme de croix de nature hémisphérique (5).

12. Articulation artificielle selon l'une quelconque des revendications précédentes, dans laquelle ladite articulation est au moins en partie recouverte d'un matériau.

13. Articulation artificielle selon la revendication 12, dans laquelle ledit matériau recouvre ladite articulation de façon à réaliser une surface continue depuis l'un desdits éléments (1) vers l'autre desdits éléments (6).

14. Articulation artificielle selon les revendications 12 ou 13, dans laquelle ledit matériau est sélectionné de façon à être d'une épaisseur appropriée en vue d'une manipulation appropriée dudit instrument.

15. Articulation artificielle selon les revendications 1 à 14, dans laquelle ladite articulation artificielle est munie d'un moyen (25, 27 ; 30, 31, 9, 18, 19 ; 20) pour régir le mouvement relatif desdits éléments.

16. Articulation artificielle selon la revendication 15, dans laquelle ledit moyen comprend un moyen destiné à déterminer le mouvement le long ou autour d'au moins un axe sélectionné (2, 3), lequel moyen comprend un moyen de limitation (9, 18, 19, 20 ; 25, 27 ; 30, 31) destiné à limiter le degré de mouvement le long ou autour dudit axe.

17. Articulation artificielle selon la revendication 16, dans laquelle ledit moyen de limitation comprend un moyen mécanique (9, 18, 19, 20) ou électromécanique (25, 27 ; 30, 31).

18. Articulation artificielle selon les revendications 1 à 17, dans laquelle ladite articulation est munie d'au moins un détecteur destiné à surveiller la position relative d'un premier élément par rapport audit autre élément et destiné à transporter ces informations vers un système d'enregistrement vidéo d'où il résulte que le mouvement relatif desdits éléments résulte en un changement correspondant des images d'enregistrement vidéo de façon à simuler les images que l'on observerait normalement pendant une procédure chirurgicale.

19. Membre artificiel comprenant l'articulation artificielle selon les revendications 1 à 18.
